# EUROPEAN PATENT APPLICATION

(11) **EP 4 592 682 A1**
(43) Date of publication of application: **30.07.2025**
(21) Application number: 23867955.9
(22) Date of filing: 23.08.2023
(51) Int. Cl.: G01N 35/00

(54) **AUTOMATED ANALYSIS DEVICE, AND METHOD FOR OPERATING AUTOMATED ANALYSIS DEVICE**

(30) Priority: 21.09.2022 JP 2022150216
(71) Applicant: Hitachi High-Tech Corporation, Minato-ku Tokyo 105-6409 (JP); F. Hoffmann-La Roche AG, 4070 Basel (CH); Roche Diagnostics GmbH, 68305 Mannheim (DE)
(72) Inventor: ONOSE Toma, Tokyo 105-6409 (JP); SHIBUYA Satoshi, Tokyo 105-6409 (JP); TAKAKURA Tatsuki, Tokyo 105-6409 (JP); DATZ Stefan, 68305 Mannheim (DE); FOESTER Elisabeth, 68305 Mannheim (DE); PAUSELIUS-FUCHS Ursula, 68305 Mannheim (DE); BISHR Bassem, 6343 Rotkreuz (CH); DEGROOT Peter, 6343 Rotkreuz (CH)
(74) Representative: MERH-IP Matias Erny Reichl Hoffmann Patentanwälte PartG mbB
(86) International application number: PCT/JP2023/030361
(87) International publication number: WO 2024/062831

(57) **Abstract**

The automatic analyzer includes: an analyzing unit 40 that analyzes a sample; and a control device 20 that controls operation of each mechanism of the analyzing unit 40. The control device 20 calculates waiting time for which a user must wait before replacing the sample or a consumable necessary for analyzing the sample based on time when the sample or the consumable is last used in an analysis schedule that has been created at a point when the control device 20 receives a replacement request for the sample or the consumable. It can be to provide an automatic analyzer capable of improving work efficiency, and a method for operating the automatic analyzer.

## Description

### Technical Field

The present invention relates to an automatic analyzer and a method for operating an automatic analyzer.

### Background Art

There is a technology of enabling, in an automatic analyzer which analyzes a biological component, a user to directly access an area in the automatic analyzer in which each consumable exists, thereby replacing the consumable, (see Patent Document 1).

### Prior Art Document

### Patent Document

Patent Document 1: JP-2008-122417-A

### Summary of the Invention

### Problem to be Solved by the Invention

According to Patent Document 1, a certain time is required from when the user requests for stop of analysis operation to replace the consumable to when the user can access the area in which the consumable exists, but this time varies depending on a combination of requested items and an analysis situation, and further differs between a sample and the consumable and between the consumables to be used. That is, the user cannot know approximate time until the replacement becomes possible and hence work efficiency is not high.

An objective of the present invention is to provide an automatic analyzer and a method for operating an automatic analyzer capable of improving work efficiency.

### Means for Solving the Problem

An automatic analyzer according to one aspect of the present invention includes: an analysis unit that analyzes a sample; and a control unit that controls operation of each mechanism of the analysis unit, and the control unit calculates waiting time for which a user must wait before replacing the sample or a consumable necessary for analyzing the sample based on the time of the last use of the sample or the consumable in an analysis schedule that has been created at a point when the control unit receives a replacement request for the sample or the consumable.

### Advantages of the Invention

According to the present invention, it is possible to provide the automatic analyzer and the method for operating an automatic analyzer capable of improving the work efficiency.

### Brief Description of Drawings

Fig. 1 is a configuration diagram of an automatic analyzer.
Fig. 2 is a schematic diagram of a system reagent flow path.
Fig. 3 is a table showing an analysis sequence.
Fig. 4 illustrates a screen of an operating device.
Fig. 5 illustrates a replacement reservation screen of the operating device.
Fig. 6 illustrates transition of a replacement reservation button on the replacement reservation screen of the operating device.
Fig. 7 illustrates a replacement screen of the operating device.
Fig. 8 is an internal block diagram of a control device.
Fig. 9 is a flowchart of processing by a calculating unit.
Fig. 10 is a flowchart of first processing by the control unit.
Fig. 11 is a flowchart of second processing by the control unit.
Fig. 12 is a sequence diagram illustrating a procedure from planning to execution of analysis operation by the control device.
Fig. 13 is a sequence diagram illustrating a procedure of processing by the operating device and the control device when a user makes a reservation for replacement of a consumable.
Fig. 14 is a sequence diagram showing a procedure of processing from when the user receives replacement permission of a consumable to when the user replaces the consumable and resumes the analysis operation.
Fig. 15 illustrates a relationship between replacement reservation operation and an analysis plan.
Fig. 16 illustrates a relationship between a replacement permission notification and an analysis plan.
Fig. 17 illustrates a modification of the replacement reservation screen.

### Modes for Carrying Out the Invention

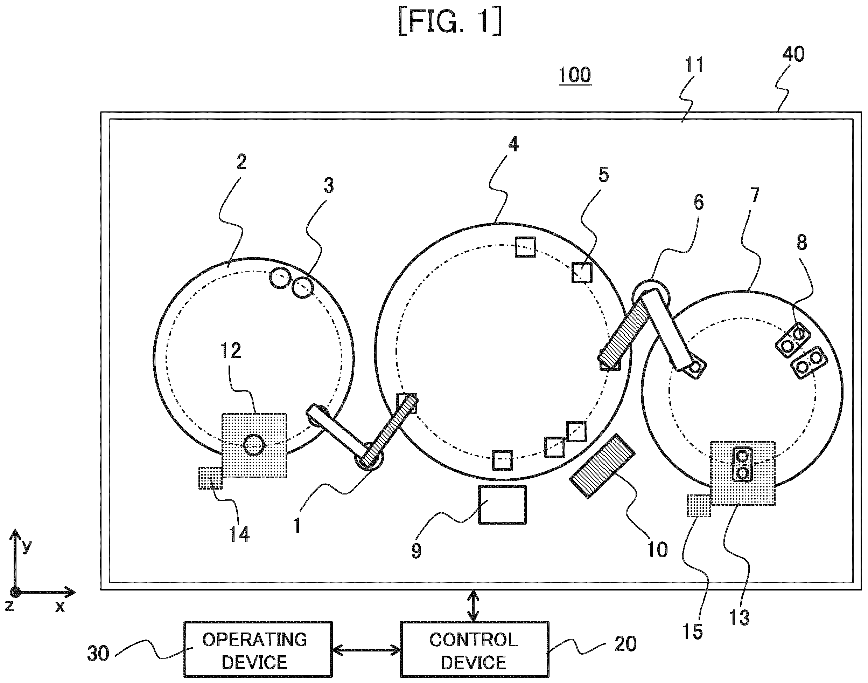

A description is now given of an embodiment of an automatic analyzer and a method for operating an automatic analyzer with reference to Fig. 1 to Fig. 17. Note that, in the drawings used in the present specification, there is such a case in which the same or corresponding component is assigned with the same or a similar reference character, and a redundant description of this component is omitted.

A description is now given of a configuration and an operation assumed for an automatic analyzer 100 with reference to Fig. 1 to Fig. 3. First, with reference to Fig. 1, an overall configuration of the automatic analyzer 100 is described.

The automatic analyzer 100 is mainly provided with an analysis unit 40 that analyzes a sample, a control device 20 that controls operation of each mechanism of the analysis unit 40, and an operating device 30 which inputs and displays various types of information. The analysis unit 40 is provided with a sample dispensing mechanism 1, a sample disc 2, a reaction disc 4, a reagent dispensing mechanism 6, a reagent disc 7, a measuring unit 9, a cleaning mechanism 10, and the like.

The sample disc 2 holds a plurality of sample containers 3 containing a sample being an analysis object. The sample disc 2 is rotatable and the shape thereof is a disc shape. The sample disc 2 transports the sample container 3 being the analysis object to a dispensing position of the sample dispensing mechanism 1 through the rotating operation.

The reaction disc 4 holds a plurality of reaction containers 5 for causing reaction and executing measurement. Note that the reaction disc 4 is rotatable, and the shape thereof is a disc shape. The reaction container 5 held by the reaction disc 4 contains a mixture obtained by mixing and reacting the sample and the reagent with each other.

The sample dispensing mechanism 1 aspirates the sample from the sample container 3 transported to a dispensing position by the sample disc 2. After that, the sample dispensing mechanism 1 dispenses the aspirated sample to the reaction container 5 held by the reaction disc 4.

The reagent disc 7 reserves a plurality of reagent containers 8 and maintains the reagent containers 8 cooled. Each reagent container 8 is filled with a first reagent, a second reagent, or a third reagent to be mixed and reacted with the sample, a reagent required for preprocessing of an analysis, or a detergent. Note that the reagent disc 7 is rotatable, and the shape thereof is a disc shape.

The reagent dispensing mechanism 6 is a mechanism which dispenses the reagent or the detergent filled in the reagent container 8 to the reaction container 5 held by the reaction disc 4. The reagent dispensing mechanism 6 dispenses the reagent or the detergent at a predetermined timing in a step including the analysis or the preprocessing therefor or the cleaning.

The measuring unit 9 measures a concentration and the like of a material being a measuring object of the mixture of the sample and the reagent during the reaction or after the reaction in the reaction container 5. A measuring method includes a method of measuring transmitted light or a method of measuring a light emission amount, and varies depending on the measuring object. Note that the measuring method is not limited and the number thereof is not limited to one and may be two or more.

The cleaning mechanism 10 includes a discharge nozzle that discharges cleaning water and the detergent, an aspiration nozzle that aspirates reacted liquid and the like, and the like. The cleaning mechanism 10 uses the cleaning water and the detergent to clean the reaction container 5.

The automatic analyzer 100 is provided with, on a top surface thereof, a top cover 11 which convers each mechanism. In addition to the top cover 11, the automatic analyzer 100 is provided with a sample lid 12 above the sample disc 2 and a reagent lid 13 above the reagent disc 7.

The sample lid 12 is provided with a sample lid locking mechanism 14, and the reagent lid 13 is provided with a reagent lid locking mechanism 15. The sample lid locking mechanism 14 and the reagent lid locking mechanism 15 can be locked and unlocked independently of each other and control whether or not the sample lid 12 and the reagent lid 13 can be opened and closed, respectively.

When the locking mechanisms 14 and 15 are unlocked, the user can open the lids 12 and 13, thereby being able to access the discs 2 and 7, respectively. The locking mechanisms 14 and 15 can be locked when the lids 12 and 13 are closed, respectively, the user cannot open and close the lids 12 and 13, after the locking, and hence the access to the disks 2 and 7 are also disabled, respectively.

The operating device 30 including a display device such as a display and an input device such as keyboard and a mouse, and the control device 20 including a storage unit, a CPU, and a memory may include a computer, and may include one computer or computers independently of each other, and a configuration thereof is not particularly limited.

The control device 20 is a portion which controls operation of each mechanism in the automatic analyzer 100 and causes information relating to the automatic analyzer 100 to be displayed on the operating device 30 to the user.

In the present embodiment, the control device 20 receives a replacement request for the sample or the consumable necessary for analyzing the sample, and calculates waiting time for which the user must wait before replacing the sample or the consumable based on time of the last use of the sample or the consumable in an analysis schedule that has been created at this point. Details thereof are described later.

The control of the operation of each device by the control device 20 is executed based on various programs recorded in a storage device. Note that pieces of control processing for the operation executed in the control device 20 may be unified into one program or may be divided into a plurality of programs or a combination thereof may be employed. Moreover, a part of or the whole of the program may be implemented by dedicated hardware or may be modularized.

The operating device 30 is provided with a user interface such as a display of a touch panel type, outputs information to the user, and receives various types of input from the user. In the present embodiment, the operating device 30 displays the information relating to the automatic analyzer 100 to the user as well as displays information relating to the waiting time for which the user must wait before replacing the sample or the consumable. Details thereof are described later.

The configuration of the automatic analyzer 100 according to the present embodiment has been described.

Note that the configuration of the automatic analyzer 100 is not limited to a single analysis module configuration as depicted in Fig. 1 and may be such a configuration that two or more of an analysis module which can measure various same or different analysis items and a preprocessing module which executes preprocessing are connected to one another by a transport device.

As "the consumable necessary for analyzing the sample" in the present embodiment, the reagent described before, a system reagent contained in an external container, and the like are exemplified, but the consumable is not limited thereto and may cover a dispensing tip and a disposable reaction container used for an immunoassay item or a mechanism which is consumed by analysis operation such as a drive motor of each mechanism and a spectroscope.

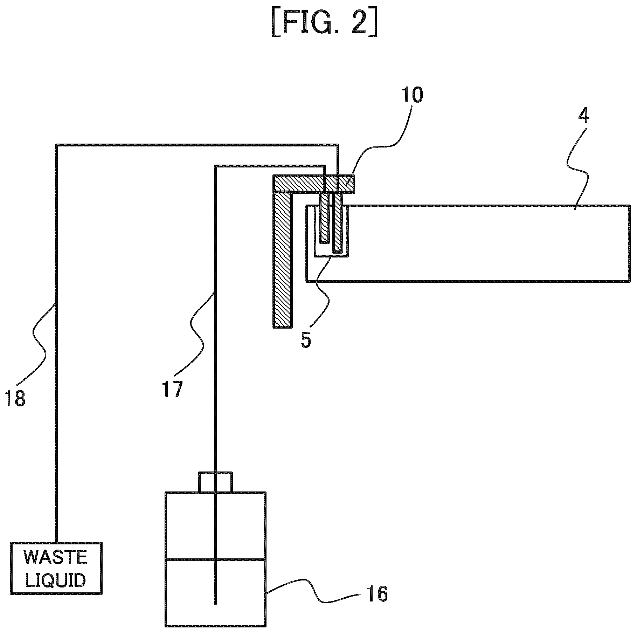

A description is now given of the system reagent provided to the automatic analyzer 100 with reference to Fig. 2. Fig. 2 is a schematic diagram of a system reagent flow path.

As the system reagent used for the automatic analyzer 100, there exists the detergent discharged by the cleaning mechanism 10 to clean the reaction container. The detergent discharged by the cleaning mechanism 10 is filled in an external detergent bottle 16. The external detergent bottle 16 is connected to a detergent flow path 17 and the detergent flow path 17 is connected to the discharge nozzle of the cleaning mechanism 10. A waste liquid flow path 18 is connected to the aspiration nozzle of the cleaning mechanism 10. The cleaning mechanism 10 discharges, into the reaction container 5, the detergent filled in the external detergent bottle 16 via the detergent flow path 17. Moreover, the cleaning mechanism 10 aspirates the detergent discharged into the reaction container 5 and processes the detergent as waste liquid via the waste liquid flow path 18.

The detergent flow path 17 can be closed and opened via the cleaning mechanism 10. The flow path is closed to enable the user to replace the detergent when the external detergent bottle 16 is to be replaced, and the flow path is opened after the replacement is completed, thereby enabling the use of the detergent.

The analysis processing (processing of dispensing the sample, mixing the reagent with the sample to react the sample, measuring the absorbance to obtain the concentration) for the sample by the automatic analyzer 100 as described above is generally executed according to the following procedure.

In the automatic analyzer 100, color development and light emission caused by reaction between a target component in the sample and the reagent are measured by the measuring unit 9, and concentration of the target component in the sample is calculated through computation processing of the control device 20. An overview of the analysis of the sample by the automatic analyzer 100 is described as follows.

The user puts the sample in the sample container 3 such as a test tube and mounts the sample container 3 to the sample disc 2. The user requests, from the operating device 30, an analysis item to be measured on the sample, thereby starting the measurement. The sample is aspired from the sample container 3 by the sample dispensing mechanism 1 in the automatic analyzer 100 and is discharged into the reaction container 5. As described above, the automatic analyzer 100 holds the reagent in the reagent container 8 in the device and uses the reagent dispensing mechanism 6 to discharge the reagent into the reaction container 5 containing the sample. Subsequently, the reaction between the sample and the reagent progresses in the reaction container 5.

In the automatic analyzer 100, signal values of the color development and the light emission of the mixture in the reaction container 5 are measured at predetermined position and timing by the measuring unit 9. The computation processing is executed in the control device 20 based on the measured signal values to compute, as a measurement result, the concentration of the target component. The reaction container 5 for which the measurement has been completed is cleaned by the cleaning mechanism 10 according to necessity and is prepared for a subsequent analysis.

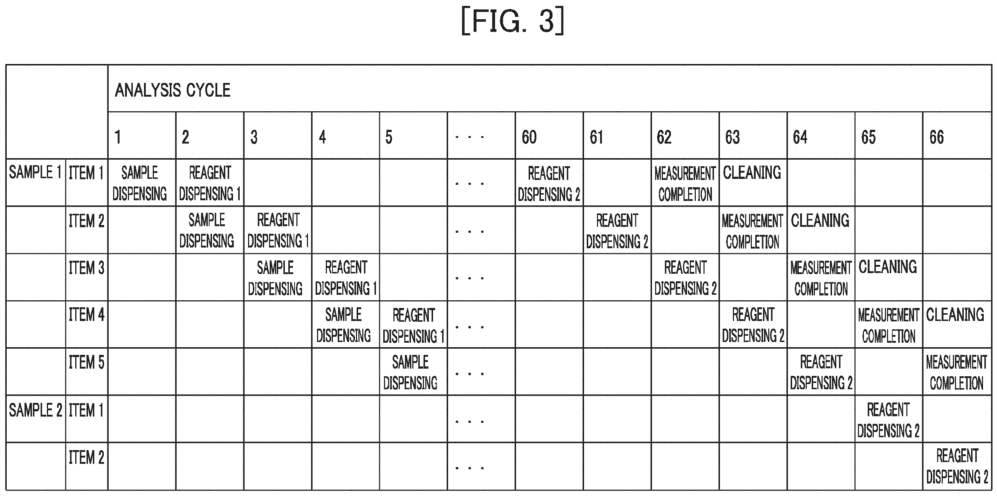

A description is now given of characteristic configuration and operation according to the present embodiment with reference to Fig. 3 and subsequent drawings.

In the analysis of the sample by the automatic analyzer 100, the reagents are sometimes dispensed for a plurality of times at different timings depending on the analysis item.

For example, there is such a case in which, after the first reagent is dispensed and the signal values are measured, after the second reagent is dispensed, the signal values are further measured, and the concentration of the target component is calculated based on the plurality of measurement results. In this case, as depicted in Fig. 3, the second reagent is dispensed into the mixture by the reagent dispensing mechanism 6 and, after that, the signal values in the reaction container 5 are measured by the measuring unit 9, thereby computing the concentration of the target component. Fig. 3 is a diagram depicting an example of a series of sequences for executing a plurality of analyses through the operation described above.

The automatic analyzer 100 holds the plurality of reaction containers 5 and hence is configured to be able to execute a plurality of analyses continuously and in parallel through the operation described above. Each mechanism executes objective operation in each analysis cycle repeated after each certain time as depicted in Fig. 3.

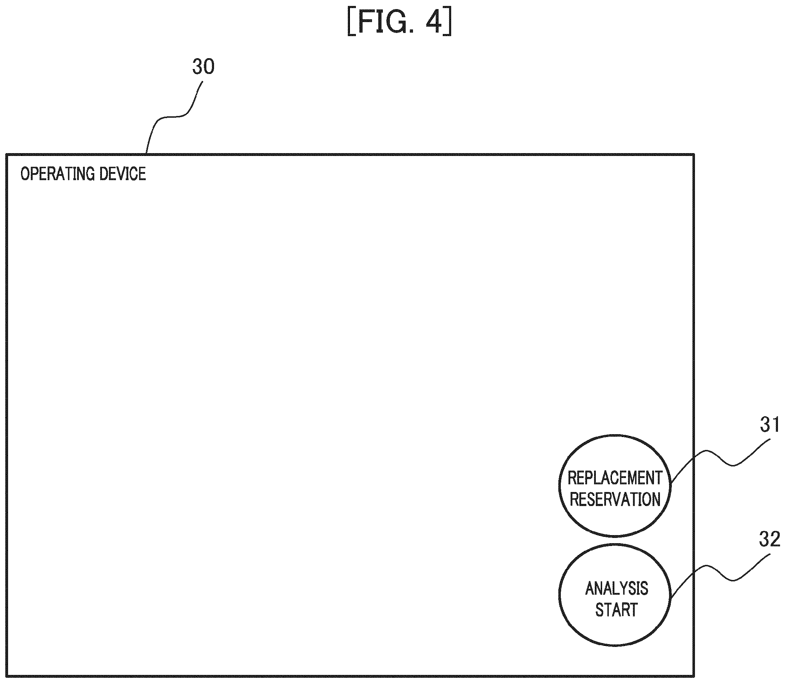

With reference to Fig. 4 to Fig. 6, a description is now given of a configuration of the operating device 30.

First, with reference to Fig. 4, a screen configuration of the operating device 30 is described. Fig. 4 is a view of a screen of the operating device.

As depicted in Fig. 4, a replacement reservation button 31 and an analysis start button 32 are displayed at a usual time on the screen displayed on the operating device 30.

When the user holds down the replacement reservation button 31, the control device 20 causes the screen to transition from the usual screen to a replacement reservation screen 33.

When the user holds down the analysis start button 32, an analysis start request is transmitted to the control device 20, and the analysis starts or a temporary stopped analysis is resumed.

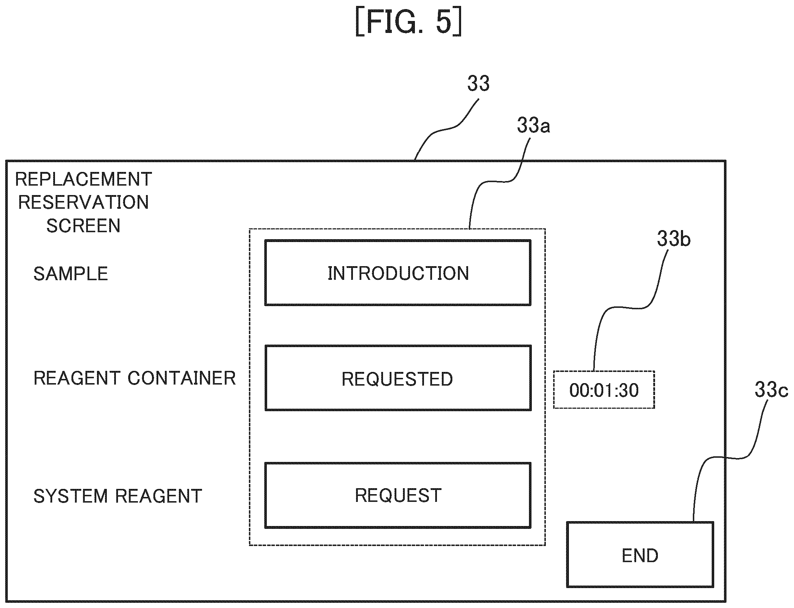

With reference to Fig. 5, a description is now given of a configuration of the replacement reservation screen 33. Fig. 5 is a view of the replacement reservation screen of the operating device.

As depicted in Fig. 5, on the replacement reservation screen 33, the replacement reservation button 33a is displayed for each of replacement objects (in Fig. 5, the sample, the reagent container, and the system reagent).

The replacement reservation button 33a is a button displayed on the operating device 30 for the user to issue the replacement request, and strings corresponding to three states are displayed on the button according to the replacement reservation situation (in Fig. 5, "request (wait for reservation)," "requested (reserved)," and "introduction (replacement permission)."

The control device 20 receives the replacement request when the control device 20 recognizes that this replacement reservation button 33a is held down and starts execution of various types of processing necessary for the replacement. At this time, the control device 20 causes remaining time (waiting time) until the replacement permission to be displayed as a remaining time display 33b around the replacement reservation button 33a held down by the user, only when the replacement reservation button 31 is in the state of "requested (reserved)."

The remaining time until the replacement permission displayed in the periphery of the replacement reservation screen 33 can be displayed in a countdown form, but the form is not limited to a form of a numerical value itself, but may be a form such as a progress bar representing a progress until the replacement permission. Moreover, not the remaining time, but a replacement permission possible time obtained from the remaining time and the current time may be displayed.

Moreover, it is assumed that the change in display of the replacement reservation button 33a is through use of the strings of "request," "requested," and "introduction," but may be a change in a button color or flickering of the button or in a composite form thereof.

For example, the control device 20 can change the display of the replacement reservation button 33a when time of the last use of the consumable comes and may employ a form such as releasing sound, changing a display color of the area of the replacement reservation button 33a or the entire screen, and flashing the display. Such a visual or auditory notification can visually be recognized from a remote place when the user is apart from the automatic analyzer, and hence is a very convenient form in such a case in which the user wants to return to the automatic analyzer when the entire automatic analyzer is in a ready state.

Further, when a plurality of replacement reservations exist, there may be provided such a form that the display is changed independently or such a form that a signal such as the change in color or sound described above is issued when all of the replacement reservations change to the replacement possible state.

An end button 33c is displayed on the replacement reservation screen 33 and when the user holds down the end button 33c, the replacement reservation screen 33 is closed.

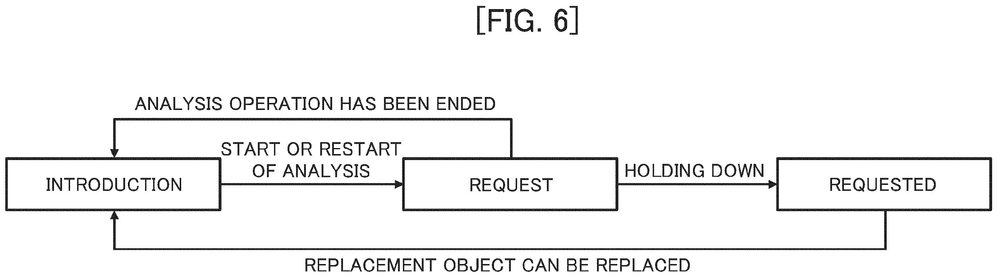

With reference to Fig. 6, a description is now given of transition of the replacement reservation button 33a. Fig. 6 is a diagram depicting transition of the replacement reservation button on the replacement reservation screen of the operating device.

On the replacement reservation button 33a, "introduction (replacement permission)" is displayed in a state other than the ongoing analysis. The display transitions to "request (wait for reservation)" at the start of the analysis. When the user holds down the replacement reservation button 33a having the display of "request (wait for reservation)," the replacement reservation request is transmitted to the control device 20, and the display of the replacement reservation button 33a transitions to "requested (reserved)."

When replacement permission information relating to the replacement object is received from the control device 20 after elapse of time, the display of the replacement reservation button 33a changes to "introduction (replacement permission)."

When the user holds down the replacement reservation button 33a having the display of "introduction (replacement permission)," a replacement start request is transmitted to the control device 20, a replacement screen 34 opens.

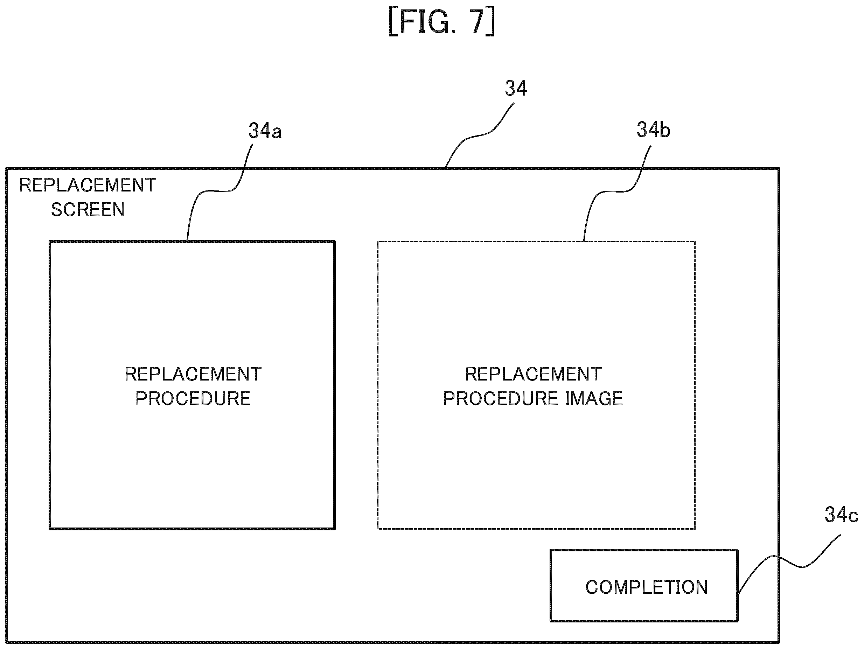

With reference to Fig. 7, a description is now given of the replacement screen 34. Fig. 7 is a view of the replacement screen of the operating device 30.

As depicted in Fig. 7, on the replacement screen 34, a replacement procedure 34a, a replacement procedure image 34b, and a completion button 34c are displayed. In the replacement procedure 34a, a replacement procedure for the replacement object is displayed as a string. In the replacement procedure image 34b, an image, a motion image, or a slideshow indicating the replacement procedure is displayed. When the user holds down the completion button 34c, the replacement screen 34 is closed and the replacement reservation screen 33 is displayed.

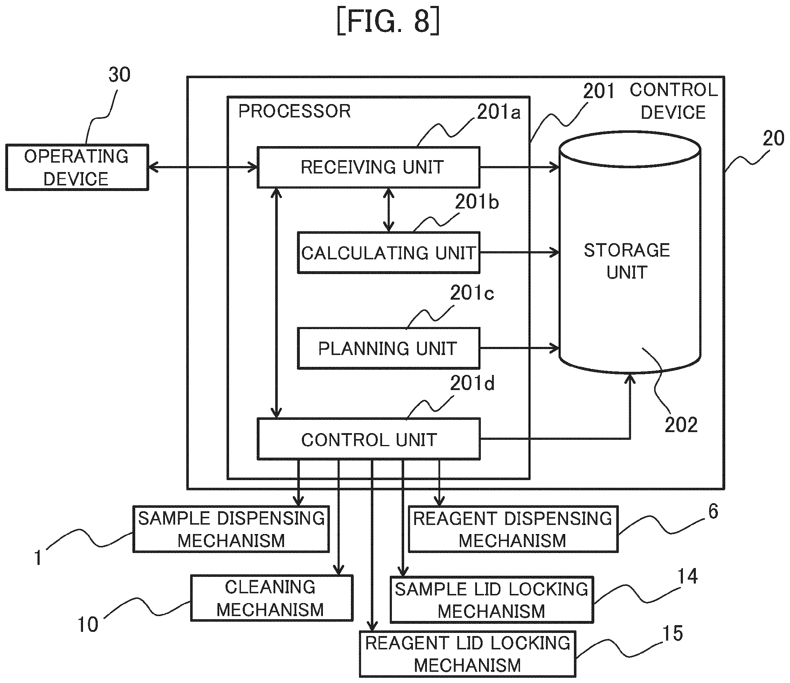

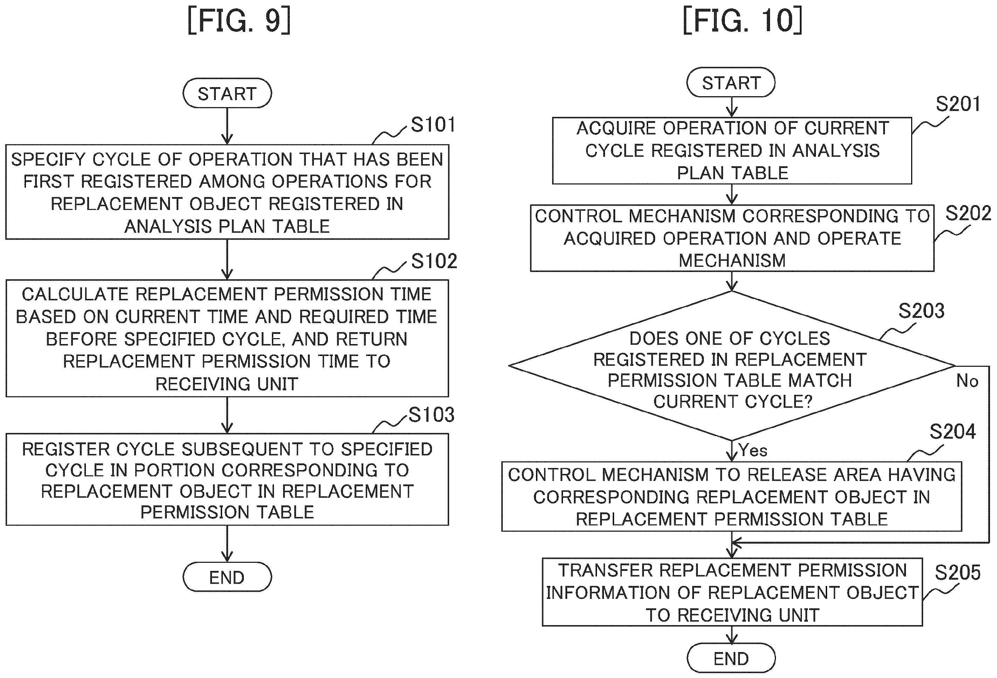

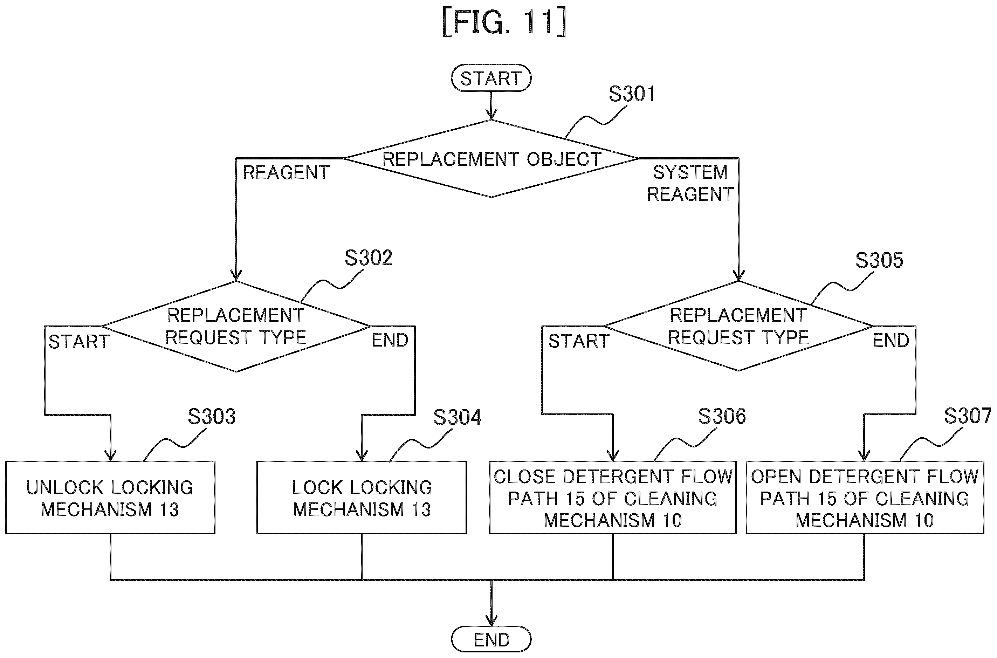

With reference to Fig. 8 to Fig. 11, a description is now given of a configuration and functions of the control device 20 provided to the automatic analyzer 100. Fig. 8 is an internal block diagram of the control device 20, Fig. 9 is a flowchart of processing by the calculating unit 201b of the control device 20, Fig. 10 is a flowchart of first processing by the control unit 201d of the control device 20, and Fig. 11 is a flowchart of second processing by the control unit 201d of the control device 20.

In Fig. 8, the control device 20 is provided with a processor 201 and a storage unit 202 (a hard disk, a memory, and the like).

The storage unit 202 is provided with an analysis plan table which is used to plan measuring items requested for each sample and mechanism operation in each analysis cycle therefor, a plan validity flag which holds validity information relating to the analysis plan, and a replacement permission table which registers the analysis cycle in which the area having a consumable for which the replacement reservation has been received is released.

The analysis plan table has a structure similar to the analysis sequence depicted in Fig. 3 and has registered mechanism operation to be executed in each cycle.

The processor 201 includes units being a receiving unit 201a, the calculating unit 201b, a planning unit 201c, and the control unit 201d.

The receiving unit 201a communicates information with the operating device 30, the calculating unit 201b, and the control unit 201d, and updates information for the storage unit 202.

When the receiving unit 201a receives the analysis start request from the operating device 30, the receiving unit 201a executes processing of setting the plan validity flag to valid.

Moreover, when the receiving unit 201a receives the replacement reservation request from the operating device 30, the receiving unit 201a executes processing of calling the calculating unit 201b, returning required time calculated by the calculating unit 201b to the operating device 30, and setting the plan validity flag to invalid.

Moreover, when the receiving unit 201a receives the replacement permission information from the control unit 201d, the receiving unit 201a executes processing of transmitting the replacement permission information to the operating device 30.

When the receiving unit 201a receives the replacement start request or a replacement end request from the operating device 30, the receiving unit 201a executes processing of calling (passing a type indicating whether the request is the replacement start request or the replacement end request to) the control unit 201d.

The receiving unit 201a, through these pieces of processing, receives the analysis start request, the replacement reservation request, the replacement start request, and the replacement end request input from the operating device 30, and notifies the operating device 30 of waiting time information and the replacement permission information.

The calculating unit 201b communicates information with the receiving unit 201a, and refers to or updates the information in the storage unit 202.

When the calculating unit 201b is called from the receiving unit 201a, the calculating unit 201b first specifies a cycle of operation that has been first registered among pieces of operation for the replacement object registered to the analysis plan table as depicted in Fig. 9 (S101). After that, the calculating unit 201b calculates replacement permission time based on current time and required time until the specified cycle, and returns the replacement permission time to the receiving unit 201a (S102). After that, the calculating unit 201b registers a cycle subsequent to the specified cycle to a portion corresponding to the replacement object in the replacement permission table (S103). As a result, the calculating unit 201b calculates, based on the analysis plan table of the storage unit 202, the time at which the replacement of the replacement object is possible and returns the calculated time information to the receiving unit 201a.

The planning unit 201c receives the information from the operating device 30, and refers to and updates the information for the storage unit 202.

The planning unit 201c starts at the end of each analysis cycle and executes processing of registering the analysis operation of each mechanism to the analysis plan table when the plan validity flag is valid, and completing the processing without the registration when the plan validity flag is invalid.

As a result, the planning unit 201c plans the mechanism operation according to the content of each analysis item and the planned mechanism operation on the basis of requested item information for each sample notified from the operating device 30 when the plan validity flag is set to valid, and registers the analysis plan to the analysis plan table of the storage unit 202.

The control unit 201d communicates information with the receiving unit 201a, refers to the information in the storage unit 202, and controls the operation of each mechanism such as the sample dispensing mechanism 1, the reagent dispensing mechanism 6, the cleaning mechanism 10, the sample lid locking mechanism 14, and the reagent lid locking mechanism 15.

The control unit 201d starts at the start of each analysis cycle, first acquires the operation of the current cycle registered to the analysis plan table as depicted in Fig. 10 (S201), and causes the mechanism corresponding to the acquired operation to operate (S202).

After that, the control unit 201d determines whether any one of the cycles registered to the replacement permission table matches the current cycle (S203), controls the mechanism to release the area having the corresponding replacement object in the replacement permission table when the matching is determined (S204), transfers the replacement permission information of the replacement object to the receiving unit 201a (S205), and completes the processing.

Meanwhile, when the control unit 201d determines that any one of the cycles registered to the replacement permission table does not match the current cycle, the control unit 201d transfers the replacement permission information of the replacement object to the receiving unit 201a (S205) and completes the processing.

As a result, control unit 201d refers to the analysis plan table of the storage unit 202 to control each mechanism to execute the mechanism operation planned for each cycle.

Moreover, in a case in which the replacement object having the replacement reservation request reaches the replacement permission cycle, the control unit 201d notifies the receiving unit 201a of the replacement permission information.

First, the control unit 201d is started by the call from the receiving unit 201a and determines whether the replacement object is the reagent or the system reagent as depicted in Fig. 11 (S301). When the control unit 201d determines that the replacement object is the reagent, the processing is caused to proceed to Step S302 and when the control unit 201d determines that the replacement object is the system reagent, the control unit 201d causes the processing to proceed to Step S305.

In Step S302, the control unit 201d determines the replacement request type (S302). When the control unit 201d determines that the replacement request type is the start type, the control unit 201d causes the processing to proceed to Step S303, unlocks the reagent lid locking mechanism 15 (S303), and completes the processing. Meanwhile, when the control unit 201d determines that the replacement request type is the end type, the control unit 201d causes the processing to proceed to Step S304, locks the reagent lid locking mechanism 15 (S304), and completes the processing.

As described above, the control device 20 unlocks the mechanism necessary for the replacement of the sample or the consumable when the waiting time has elapsed.

In Step S305, the control unit 201d determines the replacement request type (S305), and when the control unit 201d determines that the replacement request type is the start type, the control unit 201d causes the processing to proceed to Step S306, closes the detergent flow path 17 of the cleaning mechanism 10 (S306), and completes the processing. Meanwhile, when the control unit 201d determines that the replacement request type is the end type, the control unit 201d causes the processing to proceed to Step S307, opens the detergent flow path 17 of the cleaning mechanism 10 (S307), and completes the processing.

As a result, the control unit 201d controls the cleaning mechanism 10, the sample lid locking mechanism 14, and the reagent lid locking mechanism 15 according to the replacement start request or the replacement end request from the receiving unit 201a to enable the user to directly replace each consumable.

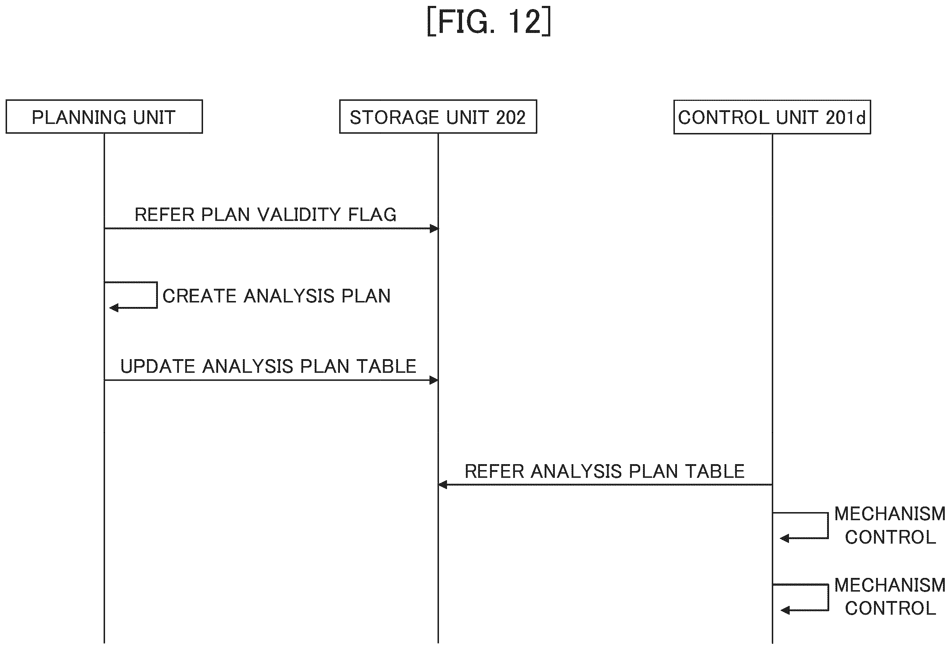

With reference to Fig. 12 to Fig. 14, a description is now given of flows of processing executed in the operating device 30 and the control device 20.

First, with reference to Fig. 12, a description is now given of operation executed by the control device 20 to plan and execute the analysis step. Fig. 12 is a sequence diagram depicting a procedure in which the control device plans and then executes the analysis operation.

As depicted in Fig. 12, the planning unit 201c starts at the end of each analysis cycle, refers to the plan validity flag in the storage unit 202 before the planning, and determines whether the plan is valid. When the planning unit 201c determines that the planning is valid, the planning unit 201 plans the operation and registers the planned operation to the analysis plan table of the storage unit 202. At the start of a subsequent analysis cycle, the control unit 201d starts, refers to the analysis plan table, and controls various mechanism according to the mechanism operation planned for this cycle. These pieces of processing are repeated for each analysis cycle, resulting in progress in analysis step.

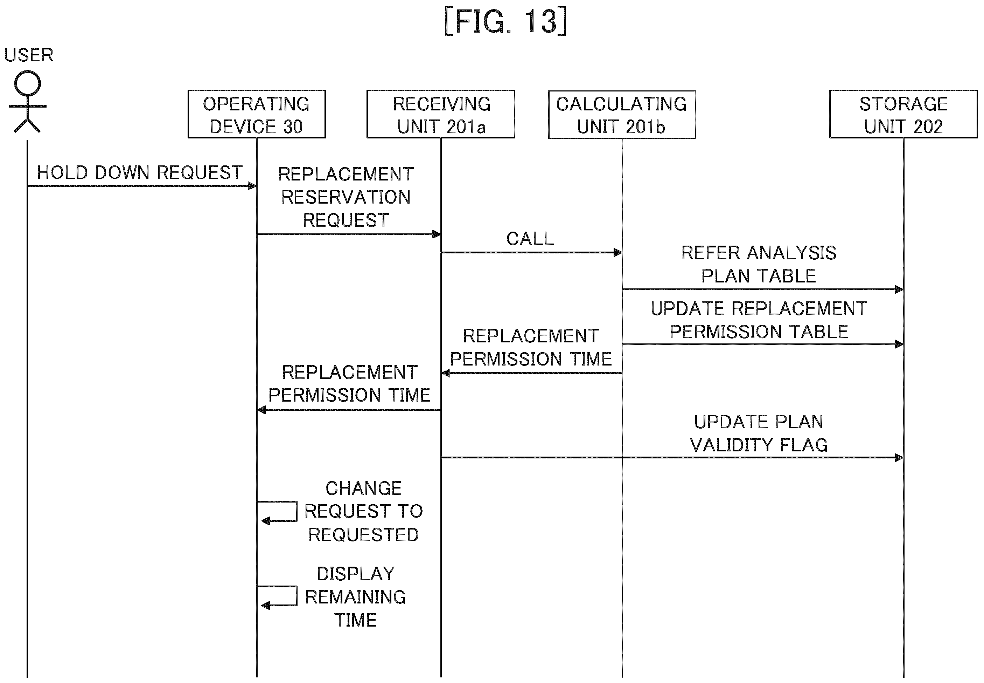

With reference to Fig. 13, a description is now given of a procedure of processing when the user makes the replacement reservation for the replacement object during the analysis operation. Fig. 13 is a sequence diagram depicting a procedure of processing of the operating device and the control device when the user makes the replacement reservation for the consumable.

As depicted in Fig. 13, when the user holds down the replacement reservation button 33a on the replacement reservation screen 33 corresponding to a specific replacement object and having the display of "request," the replacement reservation request is transmitted from the operating device 30 to the control device 20.

In the control device 20, the receiving unit 201a receives the replacement reservation request and calls the calculating unit 201b. The calculating unit 201b refers to the analysis plan table to specify the analysis cycle of the last use of the replacement object, and registers an analysis cycle subsequent to the specified analysis cycle to a portion which is out of the replacement permission table and corresponds to the replacement object.

After that, the calculating unit 201b calculates the replacement permission time based on the time until the specified analysis cycle and the current time, and returns the replacement permission time to the receiving unit 201a. After the receiving unit 201a returns the replacement permission time to the operating device 30, the receiving unit 201a updates the plan validity flag of the storage unit 202 to invalid.

When the operating device 30 receives the replacement permission time from the receiving unit 201a of the control device 20, the operating device 30 changes the "request" display of the replacement reservation button 33a which has been held down to the "requested" display, and displays the remaining time acquired based on the replacement permission time.

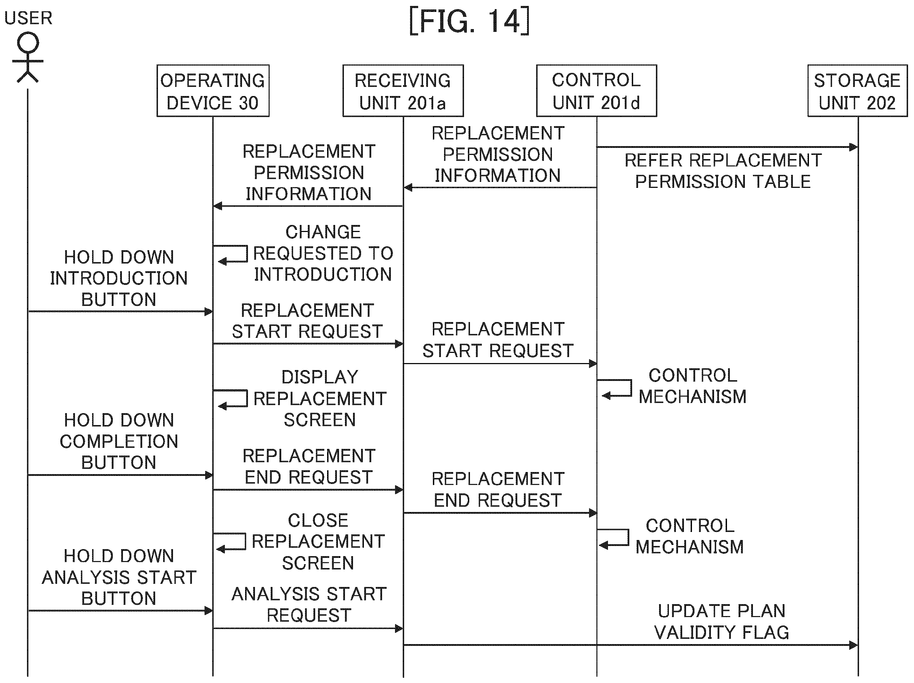

With reference to Fig. 14, a description is given of a procedure of processing in which the replacement of the replacement object for which the user made the replacement reservation during the analysis operation is permitted and the user replaces this replacement object and resumes the analysis operation. Fig. 14 is a sequence diagram depicting the procedure of the processing from when the user receives the replacement permission for the consumable to when the user resumes the analysis operation.

As depicted in Fig. 14, the control unit 201d starts at the start of each analysis cycle, executes the mechanism operation control based on the analysis plan table described before, and then refers to the replacement permission table to determine whether the current analysis cycle is the replacement permission cycle for each replacement object.

When the current analysis cycle is the replacement permission cycle, the control unit 201d calls the receiving unit 201a and notifies the receiving unit 201a of the replacement permission information. The receiving unit 201a transmits the notified replacement permission information to the operating device 30. The operating device 30 receives the replacement permission information and changes the "requested" display of the replacement reservation button 33a corresponding to the replacement object to the "introduction" display.

When the user holds down the replacement reservation button 33a having the "introduction" display at this time, the operating device 30 transmits the replacement start request to the control device 20. The receiving unit 201a receives the replacement start request in the control device 20 and calls the control unit 201d. The control unit 201d determines a mechanism of the control object and a control content according to the content of the replacement start request, and controls the object mechanism. After that, the operating device 30 displays the replacement screen 34. At this time, the user can replace the replacement object.

When the user holds down the completion button on the replacement screen 34 after the replacement of the replacement object, the replacement end request is transmitted from the operating device 30 to the control device 20. The receiving unit 201a receives the replacement end request in the control device 20 and calls the control unit 201d. The control unit 201d determines the mechanism of the control object and the control content according to the content of the replacement end request and controls the object mechanism. After that, the operating device 30 closes the replacement screen 34.

When the user holds down the analysis start button, the analysis start request is transmitted from the operating device 30 to the control device 20. In the control device 20, the receiving unit 201a receives the analysis start request and updates the plan validity flag of the storage unit 202 to valid. As a result, the planning of the analysis operation by the planning unit 201c is resumed, and the analysis operation is resumed.

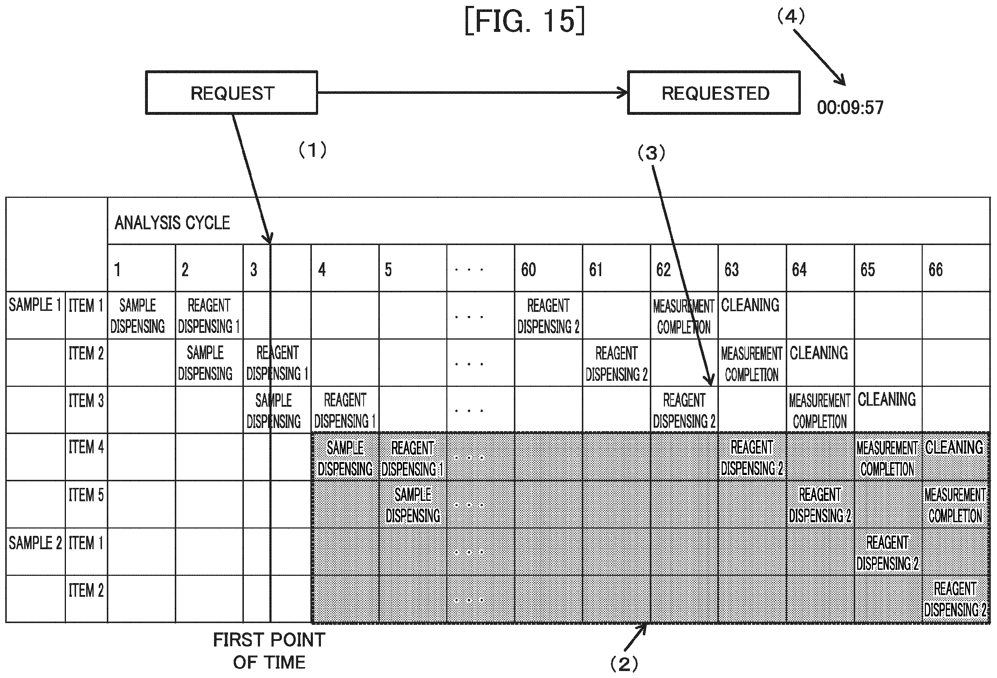

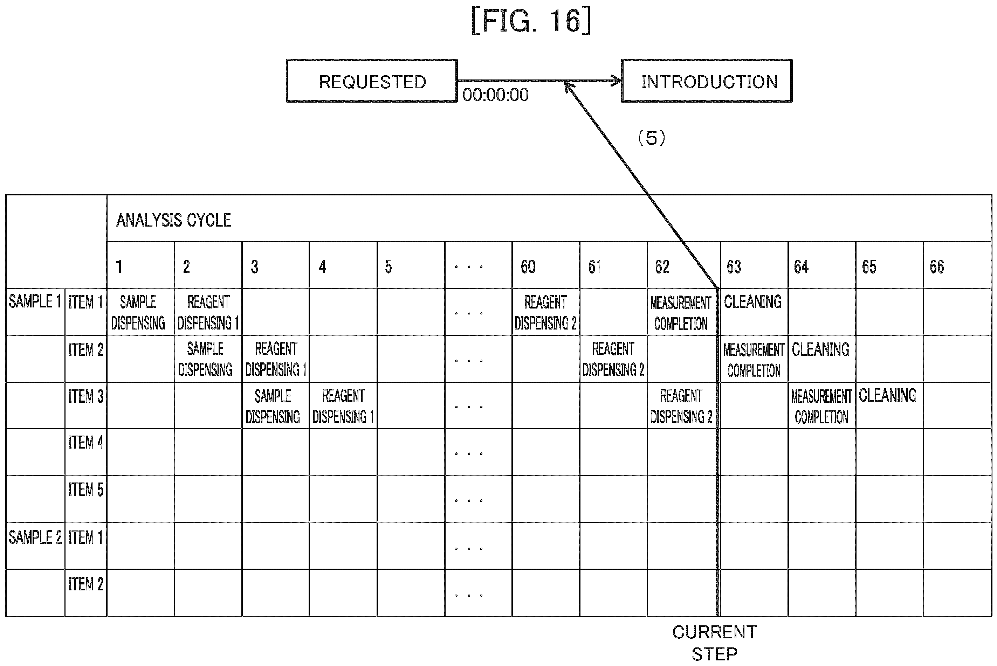

With reference to Fig. 15 and Fig. 16, a description is now given of association between the replacement operation and the analysis plan implemented by the processing described above through an example.

First, with reference to Fig. 15, a description is given of an example of the analysis plan and the behavior of the change in display after the replacement reservation button 33a having the "request" display is held down. Fig. 15 is a diagram depicting the association between the replacement reservation operation and the analysis plan.

In the example depicted in Fig. 15, it is assumed that one analysis cycle is 10 seconds, a time at 0 hours, 0 minutes, and 0 seconds being a point when three seconds has elapsed in a third analysis cycle is set to a first point of time, and the user makes the replacement reservation for the reagent container at the first point of time.

As indicated as (1) in Fig. 15, when the replacement reservation button 33a having the "request" display is held down, the analysis plan by the planning unit 201c stops. As a result, as indicates as (2) in Fig. 15, the registration of an analysis plan, which has not been started yet, to the analysis plan table is stopped and hence the planning for the analysis operation for items subject to the sample dispensing in fourth and subsequent analysis cycles is stopped.

As described above, when the control device 20 receives the replacement request, it is desired that the control device 20 temporarily stop the dispensing plan for the samples which have not been started yet, thereby being able to achieve as fast replacement work as possible.

After that, the control device 20 specifies the analysis cycle being the final use timing of the replacement object in the planned analysis registered to the analysis plan table. In this example, the replacement reservation for the reagent container is made, and hence 62nd analysis cycle in which the reagent dispensing is executed last is specified as indicated as (3) in Fig. 15.

A 63rd analysis cycle being an analysis cycle subsequent to the specified analysis cycle becomes the replacement permission cycle, 59 analysis cycles and seven seconds are reckoned from the first point of time until the replacement permission cycle starts, and hence a total of 597 seconds are the remaining time.

Thus, the time until the final use timing is calculated and displayed based on the current time and the required time of each step. In this example, as indicated as (4) in Fig. 15, the remaining time displayed adjacent to the replacement reservation button 33a which now has the "requested" display is 9 minutes and 57 seconds.

With reference to Fig. 16, a description is now given of an example of the analysis plan and the behavior of the display change after the replacement permission cycle starts. Fig. 16 is a diagram depicting the association between the replacement permission notification and the analysis plan.

As depicted in Fig. 16, when the current step is the beginning of the 63rd analysis cycle, the start of the replacement permission cycle of the replacement object is detected as a result of the start of the control unit 201d in the current step, and the "requested" display of the replacement reservation button 33a is changed to the "introduction" display as a result of the replacement permission information is notified to the operating device 30.

After that, when the user holds down the replacement reservation button 33a having the "introduction" display, the reagent lid 13 becomes openable as a result of the unlocking of the reagent lid locking mechanism 15, and hence there is brought about the state in which the user can access the reagent disc 7.

The reagent dispensing operation is not planned subsequently to this point, hence the reagent disc 7 and the reagent dispensing mechanism 6 do not operate, and the replacement work of the user and the mechanism operation do not interfere with each other.

Note that even in a case in which a plurality of the replacement requests are made, the time until the replacement becomes possible is calculated for each replacement request as depicted in Fig. 15. In this case, it is desired that the time required for other replacements be not considered, but the time may be considered.

Moreover, when the replacement requests are made for a plurality of the samples or the consumables, the control device 20 can display, on the operating device 30, types of sample or consumable in an ascending order of the waiting time.

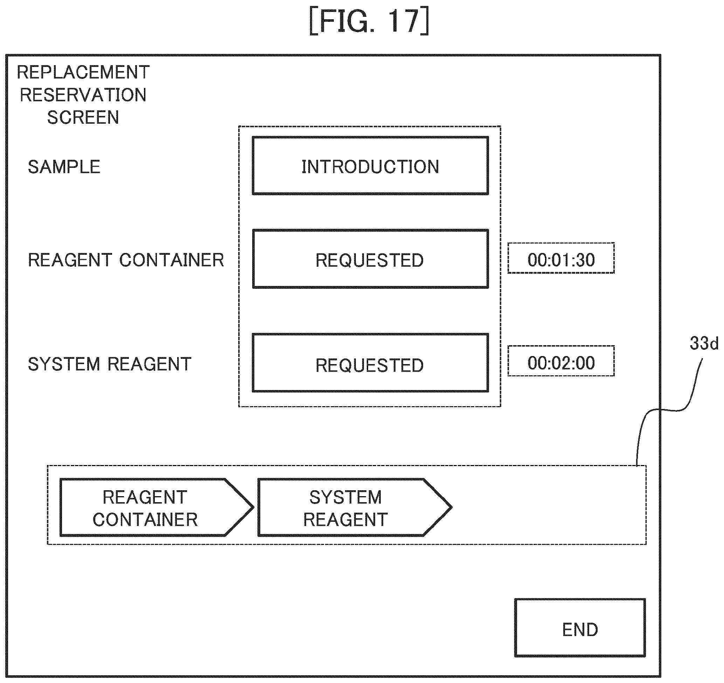

For example, as depicted in Fig. 17, there can be provided such a form that a replacement flow display area 33d is provided on the replacement reservation screen 33 and, when the replacement reservation is made for a plurality of the consumables, the consumable names are displayed in an ascending order of the waiting time, thereby enabling the user to recognize the replacement order of the consumables. As specific processing in this case, it is only required to further execute processing of comparing the remaining time with one another, thereby displaying, in the replacement flow display area 33d, the remaining times in the ascending order.

Effects of the present embodiment are now described.

The automatic analyzer 100 according to the present embodiment described above is provided with the analysis unit 40 which executes the analysis of the sample and the control device 20 which controls the operation of each mechanism of the analysis unit 40. The control device 20 receives the replacement request for the sample or the consumable necessary for the analysis of the sample and calculates, based on the time of the last use of this sample or consumable in the analysis schedule that has been created at this point, the waiting time for which the user must wait before replacing the sample or the consumable.

With this configuration, in the automatic analyzer 100 in which the user accesses the area that is inside the automatic analyzer 100 and in which each consumable exists to directly replace the consumable, when the user wants to replace the consumable during the analysis, the start of the analysis is temporarily stopped, the access to the area in which the consumable exists becomes possible for the user after this consumable is used last in the started analysis operation, and the waiting time until the access to the area becomes possible can be presented to the user. Thus, the user can know the time until the replacement becomes possible, and hence work efficiency can be improved.

Moreover, the automatic analyzer 100 is further provided with the operating device 30 which displays the information relating to the automatic analyzer 100 and the information relating to the waiting time, the control device 20 causes the information to be displayed on the operating device 30, hence the user can recognize, at a glance, the timing at which the replacement becomes possible, and work and the like can be executed according to this time. Thus, the work efficiency can further be improved.

Further, the control device 20 receives the replacement request when the control device 20 recognizes that the user holds down the replacement reservation button 33a displayed on the operating device 30 for issuing the replacement request, and displays the waiting time in the periphery of the replacement reservation button 33a held down by the user, thereby enabling easy recognition of the waiting time until the replacement of the sample or the consumable the replacement of which is requested becomes possible.

Moreover, when the control device 20 receives the replacement request, the control device 20 temporarily stops the dispensing plan for the samples which have not been started yet, thereby being able to prevent the sample from being left in the state in which the sample is dispensed.

Further, the control device 20 changes the display of the replacement reservation button 33a when the time of the last use of the consumable comes, thereby presenting, to the user, that the replacement becomes possible, and hence the user can quickly recognize that the user can start the replacement work, thereby being able to achieve more friendly specifications for the user.

Moreover, when the replacement requests are issued for a plurality of the samples or the consumables, the control device 20 displays, on the operating device 30, the types of sample or consumable in the ascending order of the waiting time, and hence recognition of how to proceed with the plurality of pieces of replacement work becomes easy, thereby being able to achieve further improvement of the workability of replacement work.

Further, when the waiting time has elapsed, the control device 20 unlocks the mechanism necessary for the replacement of the sample or the consumable, thereby being able to prevent such a situation that the unlocking occurs before the replacement becomes possible and forced replacement work is executed.

Moreover, the control device 20 causes the waiting time to be displayed in the countdown form, so that the timing at which the replacement becomes possible can be recognized at a glance.

### Others

Note that the present invention is not limited to the embodiment described above and various modifications and applications are possible. The embodiment described above is detailed for the sake of an easy-to-understand description of the present invention, and the present invention is not necessarily limited to the embodiment provided with all of the described configurations.

### List of Reference Signs

1 Sample dispensing mechanism
2 Sample disc
3 Sample container
4 Reaction disc
5 Reaction container
6 Reagent dispensing mechanism
7 Reagent disc
8 Reagent container
9 Measuring unit
10 Cleaning mechanism
11 Top cover
12 Sample lid
13 Reagent lid
14 Sample lid locking mechanism
15 Reagent lid locking mechanism
16 External detergent bottle
17 Detergent flow path
18 Waste liquid flow path
20 Control device (control unit)
30 Operating device (display unit)
31 Replacement reservation button
32 Analysis start button
33 Replacement reservation screen
33a Replacement reservation button (button)
33b Remaining time display
33c End button
33d Replacement flow display area
34 Replacement screen
34a Replacement procedure
34b Replacement procedure image
34c Completion button
40 Analysis unit
100 Automatic analyzer
201 Processor
201a Receiving unit
201b Calculating unit
201c Planning unit
201d Control unit
202 Storage unit

## Claims

1. An automatic analyzer, comprising:
an analyzing unit that analyzes a sample; and
a control unit that controls operation of each mechanism of the analyzing unit,
wherein the control unit calculates waiting time for which a user must wait before replacing the sample or a consumable necessary for analyzing the sample based on time when the sample or the consumable is last used in an analysis schedule that has been created at a point when the control unit receives a replacement request for the sample or the consumable.

2. The automatic analyzer according to claim 1, further comprising a display unit that displays to the user information on the automatic analyzer and information on the waiting time,
wherein the control unit makes the display unit to display the pieces of information.

3. The automatic analyzer according to claim 2, wherein when recognizing that the user presses a button to issue the replacement request displayed on the display unit, the control unit receives the replacement request and displays the waiting time in the periphery of the button pressed by the user.

4. The automatic analyzer according to claim 1, wherein the control unit suspends an unstarted dispensing plan for the sample when receiving the replacement request.

5. The automatic analyzer according to claim 3, wherein the control unit changes display of the button when time of last use of the consumable comes.

6. The automatic analyzer according to claim 2, wherein when a replacement request is issued for a plurality of the samples or the consumables, the control unit displays types of the samples or the consumables on the display unit in ascending order of the waiting time.

7. The automatic analyzer according to claim 1, wherein when the waiting time has elapsed, the control unit unlocks a mechanism necessary to replace the sample or the consumable.

8. The automatic analyzer according to claim 3, wherein the control unit displays the waiting time in a countdown manner.

9. A method for operating an automatic analyzer analyzing a sample, wherein waiting time for which a user must wait before replacing the sample or a consumable necessary for analyzing the sample is calculated based on time when the sample or the consumable is last used in an analysis schedule that has been created at a point when a replacement request for the sample or the consumable is received.
